# EUROPÄISCHE PATENTSCHRIFT

(11) **EP 1 201 673 B1**
(45) Veröffentlichungstag und Bekanntmachung des Hinweises auf die Patenterteilung: **14.01.2004**
(21) Anmeldenummer: 01123115.6
(22) Anmeldetag: 27.09.2001
(51) Int. Cl.: C07F 9/00, C07F 9/46, C07F 9/48, C07F 9/50

(54) **Bidentate Organophosphorliganden und ihre Verwendung in der Katalyse**
Bidentate organophosphorous ligands and their use in catalysis
Ligands organophosphores et leur utilisation en catalyse

(30) Priorität: 25.10.2000 DE 10052868
(43) Veröffentlichungstag der Anmeldung: 02.05.2002
(73) Patentinhaber: Degussa Aktiengesellschaft, 40474 Düsseldorf (DE)
(72) Erfinder: Monsees, Axel, Dr., 60487 Frankfurt (DE); Dingerdissen, Uwe, Dr., 64342 Seeheim-Jugenheim (DE); Laschat, Sabine, Prof. Dr., 38106 Braunschweig (DE); Sell, Thorsten, 45468 Mühlheim a. d. Ruhr (DE)
(74) Vertreter: Ackermann, Joachim, Dr.

(56) Entgegenhaltungen:
- WO-A-96/38456
- US-A- 5 175 335
- KOMAROV I V ET AL: "Synthesis of chiral functionalized phosphine ligands based on camphor skeleton" TETRAHEDRON: ASYMMETRY, ELSEVIER SCIENCE PUBLISHERS, AMSTERDAM, NL, Bd. 8, Nr. 3, 6. Februar 1997 (1997-02-06), Seiten 435-445, XP004094316 ISSN: 0957-4166
- SELL, T.; LASCHAT, S.; DIX, I.; JONES, P.G.: "A Concise Ex Chiral Pool Approach to Novel Bidentate Camphane Phosphane Ligands" EUROPEAN JOURNAL OF ORGANIC CHEMISTRY, Bd. 2000, Nr. 24, 4. Dezember 2000 (2000-12-04), Seiten 4119-4124, XP002183402 Weinheim

## Beschreibung

Die vorliegende Erfindung beinhaltet neue unsymmetrische chirale Diphosphine und Phosphin-Phosphinite und deren Synthese sowie Komplexe dieser Verbindungen mit Metallen der Gruppen Vllb VIIIb und Ib des Periodensystems sowie deren Verwendung als Katalysatoren für enantioselektive Transformationen, insbesondere für Hydrierungen.

Trisubstituierte Organophosphorverbindungen haben große Bedeutung als Liganden in der homogenen Katalyse. Durch Variation der Substituenten am Phosphor in solchen Verbindungen lassen sich die elektronischen und sterischen Eigenschaften des Phosphorliganden gezielt beeinflussen, so daß Selektivität und Aktivität bei homogen-katalytischen Prozessen gesteuert werden können. Enantiomerenangereicherte chirale Liganden werden in der asymmetrischen Synthese bzw. asymmetrischen Katalyse eingesetzt, hier kommt es wesentlich darauf an, daß die elektronischen *und* die stereochemischen Eigenschaften des Liganden auf das jeweilige Katalyseproblem optimal abgestimmt sind. Es besteht somit ein großer Bedarf an chiralen Liganden, die sich stereochemisch und elektronisch unterscheiden, um den für eine bestimmte asymmetrische Katalyse optimalen "maßgeschneiderten" Liganden aufzufinden.

Die Strukturvielfalt der bisher bekannten Phosphorliganden ist sehr groß. Die Gliederung dieser Liganden kann beispielsweise nach Stoffklassen erfolgen, und Beispiele für solche Stoffklassen sind Trialkyl- und Triarylphosphine, Phosphite, Phosphinite, Phosphonite, Aminophosphane usw. Diese Einteilung nach Stoffklassen ist insbesondere nützlich, um die elektronischen Eigenschaften der Liganden zu beschreiben.

Darüber hinaus ist eine Klassifizierung von Phosphorliganden nach ihren Symmetrieeigenschaften oder nach der Zähnigkeit der Liganden möglich. Diese Strukturierung trägt insbesondere der Stabilität, Aktivität und Stereoselektivität von Metallkomplexen mit Phosphorliganden als Katalysatorvorstufen oder als Katalysatoren Rechnung. Neben den weit verbreiteten C₂-symmetrischen bidentaten Ligandsystemen wie DUPHOS, DIPAMP, BINAP oder DEGUPHOS rücken unsymmetrische bidentate Organophosphorliganden immer mehr in den Fokus in der asymmetrischen Katalyse. Wichtige Beispiele sind die große Klasse der vielseitig einsetzbaren chiralen Ferrocenylphosphinliganden wie z.B. JOSIPHOS, DPPM, die Bisphosphinitliganden wie CARBOPHOS, die besonders in der asymmetrischen Hydrierung von Olefinen und Iminen erfolgreich eingesetzt werden, oder die Phosphin-Phosphit-Liganden wie BINAPHOS oder BIPHEMPHOS, die in der asymmetrischen Hydroformylierung von Olefinen erfolgreich eingesetzt werden.

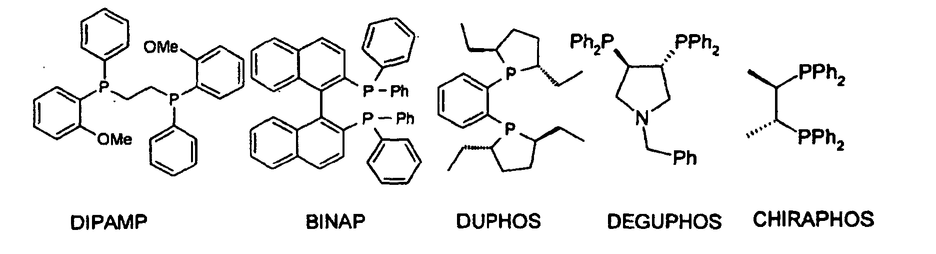

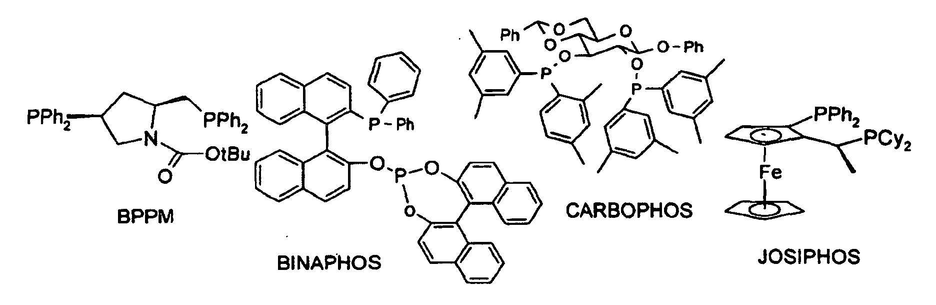

Ein wichtiger Aspekt des Erfolges dieser Verbindungsklassen wird der Schaffung einer besonders asymmetrischen Umgebung des Metallzentrums durch diese Ligandsysteme zugeschrieben. Um eine solche Umgebung für eine effektive Übertragung der Chiralität zu nutzen, ist es vorteilhaft, die Flexibilität des Ligandsystems als inhärente Limitierung der asymmetrischen Induktion zu kontrollieren.

Nachteilig bei den bisher bekannten chiralen Phosphorligandsystemen, wie sie z. B. in Komarov, I. V. et al; Tetrahedron: Asymmetry, Elsevier Science Publishers, Amsterdam NL, Bd. 8, Nr. 3, 1997, S. 435-445 beschrieben sind, ist zum einen der hohe präparative Aufwand bei ihrer Synthese und zum anderen die nur eingeschränkte Möglichkeit der Variation der Eigenschaften eines gegebenen Ligandgrundgerüstes, z. B. durch die Einführung unterschiedlichster Substituenten.

Der vorliegenden Erfindung liegt die Aufgabe zugrunde neuartige, unsymmetrische, bidentate und chirale Phosphorligandsystemen, die sich einfach in ihren sterischen und elektronischen Eigenschaften über einen außergewöhnlich weiten Raum variieren lassen, bereitzustellen.

Die Aufgabe wird durch eine Klasse chiraler, unsymmetrischer bidentater Organophosphorverbindungen der Formel (I) gelöst, wobei die erfindungsgemäßen Verbindungen ein chirales bicycloaliphatisches Grundgerüst enthalten.

Die vorliegende Erfindung betrifft somit Verbindungen der allgemeinen Formel (I), 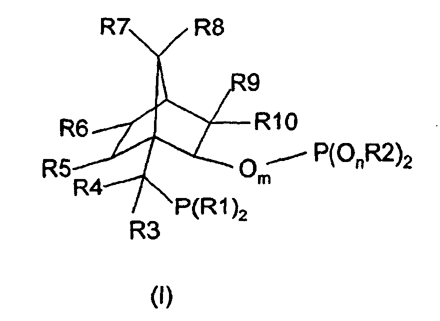 worin m und n unabhängig voneinander 0 oder 1 sein können und
- R1-R2: unabhängig voneinander für einen Rest ausgewählt aus der Gruppe C₁-C₂₄ Alkyl, C₃-C₈ Cycloalkyl, wobei der Cyclus 1-2 Heteroatome, ausgewählt aus der Gruppe N, O, S, enthalten kann, C₆-C₁₄ Aryl, Phenyl, Naphthyl, Fluorenyl, C₂-C₁₃ Heteroaryl, wobei die Zahl der Heteroatome, ausgewählt aus der Gruppe N, O, S, 1-4 betragen kann, stehen.
Die cyclischen aliphatischen oder aromatischen Reste sind bevorzugt 5 bis 6 gliedrige Ringe.
Die vorgenannten Reste selbst können jeweils ein- oder mehrfach substituiert sein. Diese Substituenten können dabei unabhängig voneinander Wasserstoff, C₁-C₂₀ Alkyl, C₂-C₂₀ Alkenyl, C₁-C₁₀ Haloalkyl, C₃-C₈ Cycloalkyl, C₂-C₉ Heterocycloalkyl, C₆-C₁₀Aryl, Phenyl, Naphthyl, Fluorenyl, C₂-C₆ Heteroaryl, wobei die Zahl der Heteroatome, insbesondere aus der Gruppe N, O, S, 1-4 betragen kann, C₁-C₁₀ Alkoxy, bevorzugt OMe, C₁-C₉ Trihalomethylalkyl, bevorzugt Trifluormethyl und Trichlormethyl, Halogeno, besonders Fluoro und Chloro, Nitro, Hydroxy, Trifluormethylsulfonato, Oxo, Amino, C₁-C₈ substituierte Amino der Formen NH-Alkyl-C₁-C₈, NH-Aryl-C₅-C₆, N-Alkyl₂-C₁-C₈, N-Aryl₂-C₅-C₆, N-Alkyl₃-C₁-C₈⁺, N-Aryl₃-C₅-C₆⁺, NH-CO-Alkyl-C₁-C₈, NH-CO-Aryl-C₅-C₆, Cyano, Carboxylato der Formen COOH und COOQ wobei Q entweder ein einwertiges Kation oder C₁-C₈-Alkyl darstellt, C₁-C₆-Acyloxy, Sulfinato, Sulfonato der Formen SO₃H und SO₃Q, wobei Q entweder ein einwertiges Kation, C₁-C₈-Alkyl oder C₆-Aryl darstellt, Phosphato der Formen PO₃H₂, PO₃HQ und PO₃Q₂, wobei Q entweder ein einwertiges Kation, C₁-C₈-Alkyl oder C₆-Aryl darstellt, Tri- C₁-C₆ Alkylsilyl, besonders SiMe₃ sind, und/oder wobei zwei Reste R¹ oder zwei Reste R² miteinander verbrückt sein können, wobei bevorzugt ein 4-8 gliedriger Zyklus gebildet wird, der gegebenenfalls mit linearen oder verzweigten C₁-C₁₀ alkyl, C₆ -aryl, benzyl, C₁-C₁₀ alkoxy, hydroxy oder benzyloxy, substituiert ist.
- R3-R10: unabhängig voneinander für ein Wasserstoffatom oder einen Rest ausgewählt aus der Gruppe C₁-C₂₄Alkyl, C₁-C₁₀-Haloalkyl, C₃-C₈ Cycloalkyl, C₃-C₈ Cycloalkenyl, wobei der Cyclus auch 1-2 Heteroatome enthalten kann, ausgewählt aus der Gruppe N, O, S, C₆-C₁₄ Aryl, Phenyl, Naphthyl, Fluorenyl, C₂-C₁₃ Heteroaryl, wobei die Zahl der Heteroatome, ausgewählt aus der Gruppe N, O, S, 1-4 betragen kann, steht.
Die cyclischen aliphatischen oder aromatischen Reste sind hier bevorzugt 5 bis 7 gliedrige Ringe.
Die vorgenannten Gruppen können selbst jeweils ein- oder mehrfach substituiert sein. Diese Substituenten können dabei unabhängig voneinander Wasserstoff, C₁-C₂₀ Alkyl, C₂-C₂₀ Alkenyl, C₃-C₈ Cycloalkyl, C₃-C₈ Cycloalkenyl, C₂-C₉ Heteroalkyl, C₁-C₉ Heteroalkenyl, C₆-C₁₀Aryl, C₁-C₁₀-Haloalkyl, Phenyl, Naphthyl, Fluorenyl, C₂-C₆ Heteroaryl, wobei die Zahl der Heteroatome, insbesondere aus der Gruppe N, O, S, 1-4 betragen kann,
C₁-C₁₀ Alkoxy, Trichlormethyl, Fluoro, Oxo, Amino, C₁-C₈ substituierte Amino der Formen der Formen N-Alkyl₂-C₁-C₈, N-Aryl₂-C₅-C₆, N-Alkyl₃-C₁-C₈⁺, N-Aryl₃-C₅-C₆⁺ sein kann,
und wobei R5 und R6 so miteinander verbrückt sein können, daß eine 5-7 gliedrige cyclische aromatische oder aliphatische Verbindung vorliegt.
- P: ist ein dreiwertiger Phosphor.

Die Erfindung betrifft ferner Komplexverbindungen, die einen chiralen bidentaten Organophosphorliganden der Formel (I) mit mindestens einem Metall enthalten. Solche Komplexverbindungen sind durch einfaches Zusammengeben der erfindungsgemäßen Organophosphorverbindungen mit Metallkomplexprecursoren in Lösung erhältlich.

Bevorzugt steht für
- R¹-R²,: unabhängig voneinander für einen Rest ausgewählt aus der Gruppe C₁-C₆Alkyl, C₅-C₆ Cycloalkyl, C₆ Aryl, Phenyl, Naphthyl, C₄-C₅ Heteroaryl, wobei die Zahl der Heteroatome, ausgewählt aus der Gruppe N, O, S, 1 beträgt, wobei die vorgenannten aromatischen oder heteroaromatischen Gruppen selbst jeweils ein- bis dreifach substituiert sein können. Diese Substituenten können dabei unabhängig voneinander Wasserstoff, C₁-C₆ Alkyl, C₂-C₄ Alkenyl, C₁-C₆ Haloalkyl, C₂-C₆ Heteroalkyl, C₆Aryl, Phenyl, Naphthyl, Fluorenyl, C₃-C₅ Heteroaryl, wobei die Zahl der Heteroatome aus der Gruppe N, O, S, 1-2 betragen kann, C₁-C₆ Alkoxy, bevorzugt OMe, C₁-C₉ Trihalomethylalkyl, bevorzugt Trifluormethyl und Trichlormethyl, Halogeno, besonders Fluoro und Chloro, Nitro, Hydroxy, Trifluormethylsulfonato, Oxo, Amino, C₁-C₆ substituierte Amino der Formen NH₂, NH-Alkyl-C₁-C₆, NH-Aryl-C₆, N-Alkyl₂-C₁-C₆, N-Aryl₂-C₆, N-Alkyl₃-C₁-C₆⁺, N-Aryl₃-C₆⁺, NH-CO-Alkyl-C₁-C₆, NH-CO-Aryl-C₆, besonders NMe₂, NEt₂, Cyano, Carboxylato der Formen COOH und COOQ wobei Q entweder ein einwertiges Kation oder C₁-C₄-Alkyl darstellt, C₁-C₆-Acyloxy, Sulfinato, Sulfonato der Formen SO₃H und SO₃Q wobei Q entweder ein einwertiges Kation, C₁-C₄-Alkyl oder C₆-Aryl darstellt, Phosphato der Formen PO₃H₂, PO₃HQ und PO₃Q₂ wobei Q entweder ein einwertiges Kation, C₁-C₄-Alkyl oder C₆-Aryl darstellt, Tri- C₁-C₆ Alkylsilyl, besonders SiMe₃ sind,

Vorzugsweise weist das erfindungsgemäße Ligandsystem in R¹-R¹⁴ Alkyl, Alkenyl, Cycloalkyl, Alkoxy, Trialkylsilyl oder/und Dialkylaminogruppen unabhängig voneinander auf, die jeweils 1 bis 20, insbesondere 1 bis 6 Kohlenstoffatome enthalten.

Aus der Gruppe der Alkylsubstituenten seien bevorzugt genannt Methyl, Ethyl, n-Propyl, 1-Methylethyl, n-Butyl, 1-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, n-Heptyl, n-Octyl, n-Nonyl, n-Decyl.

Unter den cyclischen Alkylsubstituenten sind besonders bevorzugt substituierte und unsubstituierte Cyclopentyl-, Cyclohexyl-, Cycloheptylreste.

Als Alkenylreste sind bevorzugt Vinyl, Propenyl, Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 1-Heptenyl, 2-Heptenyl, 1-Octenyl oder 2-Octenyl genannt. Unter den cyclischen Alkenylsubstituenten sind besonders bevorzugt Cyclopentenyl, Cyclohexenyl, Cycloheptenyl und Norbornyl.

Unter Arylsubstituenten in R¹-R² sind besonders bevorzugt 2-Alkylphenyl, 3-Alkylphenyl, 4-Alkylphenyl, 2,6-Dialkylphenyl, 3,5-Dialkylphenyl, 3,4,5-Trialkylphenyl, 2-Alkoxyphenyl, 3-Alkoxyphenyl, 4-Alkoxyphenyl, 2,6-Dialkoxylphenyl, 3,5-Dialkoxyphenyl, 3,4,5-Triialkoxyphenyl, 3,5-Dialkyl-4-Alkoxyphenyl, 3,5-Dialkyl-4-dialkylaminophenyl, 4-Dialkylamino, wobei die vorgenannten Alkyl- und Alkoxygruppen jeweils vorzugsweise 1 bis 6 Kohlenstoffatome enthalten, 3,5-Trifluormethyl, 4-Trifluormethyl, 2-Sulfonyl, 3-Sulfonyl, 4-Sulfonyl, ein bis vierfach halogenierte Phenyl und Naphtyl.

Bevorzugte Halogensubstituenten sind F, Cl und Br.

Alle Haloalkyl- oder/und Haloarylgruppen weisen vorzugsweise die allgemeinen Formeln CHal₃, CH₂CHal₃, C₂Hal₅ auf, wobei Hal insbesondere für F, Cl und Br stehen kann. Besonders bevorzugt sind Haloalkyl- oder/und Haloarylgruppen der Formeln CF₃, CH₂CF₃, C₂F₅.

Schließlich sind Ligandsysteme der Formel (I) als optisch aktive Ligandsysteme bevorzugt, bei denen ein Enantiomer angereichert ist. Besonders bevorzugt sind Ligandsysteme, bei denen die Enantiomerenanreicherung 90 %, insbesondere 99 % übersteigt.

Die erfindungsgemäße Klasse von bidentaten Organophosphorverbindungen besitzt ein einfach vielseitig modifizierbares, chirales Liganden-Grundgerüst, das sich in Bezug auf seine sterischen und elektronischen Eigenschaften durch die leichte Einführbarkeit unterschiedlichster Substituenten sehr breit variieren läßt. Organophosphorverbindungen der Formel (I) sind in der Lage in metallorganischen Komplexen am Metallzentrum eine hochasymmetrische Koordinationssphäre mit unabhängig voneinander modifizierbaren Organophosphordonoren zu schaffen und somit eine effektive asymmetrische Induktion zu ermöglichen. Zusätzlich können über die einfache Einführbarkeit unterschiedlichster Substituenten in die Organophosphorliganden die Flexibilität der Koordinationssphäre des Komplexes sterisch kontolliert werden.
Damit kann für Verbindungen der Formel (I) ein breites Spektrum von Anwendungen erschlossen werden, da die zweizähnigen Phosphorligandenliganden sterisch und elektronisch, je nach katalystischem Syntheseverfahren durch die Einführung geeigneter Substituenten optimierbar sind.

Gleichzeitig zeichnen sich die erfindungsgemäßen Verbindungen im Gegensatz zu vielen etablierten Ligandsystemen durch eine besonders einfache synthetische Zugänglichkeit in großer Variationsbreite ausgehend von einfachen Edukten aus. Dies macht die Liganden der vorliegenden Erfindung problemlos industriell herstellbar.

Für die Synthese von Verbindungen der Formel (I) stehen unterschiedliche Verfahren zur Verfügung, wobei von leicht zugänglichen Edukten ausgegangen wird.

Erfindungsgemäße Phosphorverbindungen aus der Klasse der Phosphin-Phosphinite lassen sich z. B. wie folgt herstellen:

Ausgehend von einem 10-Camphersulfonsäure-Derivat kann im wäßrigen basischen Milieu zunächst das Salz des Camphersulfonsäure-Derivats hergestellt werden, das in Gegenwart eines Phosphortrihalogenid den Sulfonsäurerest durch einen Halogenidrest substituiert. In einem alternativen Verfahren erfolgt die Substitution des Sulfonsäurerests durch einen Halogenidrest in einer Einschtitt-Synthese in Gegenwart von molekularem Halogen und PR_{3.} Bevorzugte Phosphortrihalogenide sind PBr₃ und Pl₃, bevorzugte molekulare Halogene sind Br₂ und l₂. Eine anschließende Reduktion liefert das entsprechende Isoborneol-Derivat.
In weiteren Verfahrensschritten wird die Hydoxygruppe des Campherderivates silyliert und anschließend mit einem Alkaliphosphin AP(R1)₂ in 10-Stellung unter Substiution des Halogenrestes phosphiniert. Die Phosphingruppe wird durch Zugabe eines Boranadduktes geschützt. Entschützung der Hydroxygruppe erfolgt mit gängigen Verfahren, wie z. B. durch Zugabe von Tetrabutylamoniumfluorid (TBAF). Danach erfolgt im basischen die Phosphinierung der Hydroxygruppe unter Zugabe eines Phosphinhalogenids HalP(R2)₂. Die neu eingeführte zweite phosphorhaltige Gruppe kann ebenfalls durch Zugabe eines Boranadduktes geschützt werden. Die Abspaltung der Boranschutzgruppen erfolgt unter Verwendung einer Stickstoffbase. Es werden die erfindungsgemäße Phosphin-Phosphinite erhalten.

Die Herstellung der Diphosphine kann ausgehend von den entsprechenden Phosphin-Phosphiniten durch Umlagerung der Phosphinitgruppe zum Phosphinoxid in der Wärme und anschließender Reduktion zum Diphosphin erfolgen.

Die Auswahl eines entsprechenden Darstellungsverfahrens ist abhängig von der Verfügbarkeit der entsprechenden Edukte und vom gewünschten Substitutionsmuster.

Die eben dargestellten Verfahren werden im folgenden anhand allgemeiner bevorzugter Verfahrensbeispiele näher erläutert.

Einfach substituierte bicyclische Grundgerüste sind aus dem *chiral* pool zugänglich. Das 10-Bromocampher wird in Anlehnung an Literaturvorschriften in einem dreistufigen Prozeß, wie oben beschrieben, analog den Vorschriften ((a) F. Dallacker, I. Alroggen, H. Krings, B. Laurs, M. Lipp, *Liebigs Ann. Chem.* **1961,** *647,* 23-36; (b) F. Dallacker, K. Ullrich, M. Lipp, *Liebigs Ann. Chem.* **1963,** *667,* 50-55; (c) N. Proth, *Rev. Tech. Lux* **1976,** 4, 195-199) hergestellt.

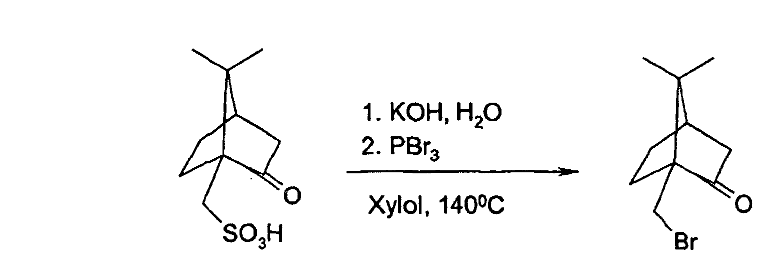

Vorteilhafter ist eine einstufige Synthese des 10-lodocampher aus 10-Camphersulfonsäure (S. Oae, H. Togo, *Bull. Chem. Soc. Jpn.* **1983**, 56, 3802-3812), gefolgt von einer selektiven Reduktion der Carbonylgruppe zum lodoalkohol mit Lithiumaluminiumhydrid.

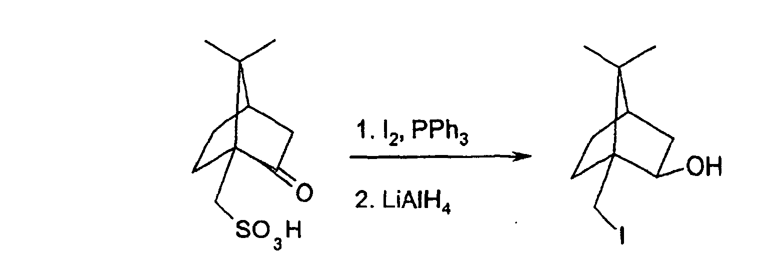

Die freie Hydroxygruppe wird durch Zugabe von Et₃SiCl mit einer Silyl-Schutzgruppe in Gegenwart einer Base geschützt, anschließend wird die Seitenkette mit Lithiumdialkyl- oder -arylphosphinen phosphiniert. Dabei können alle oben aufgeführten Reste R1 durch die Wahl eines geeigneten Alkaliphosphins selektiv eingeführt werden. Das Phosphin wird mit einem Boran-THF-Addukt als Boran-Komplex abgefangen und nach einer Desilylierung mit TABF wird das Hydroxyphosphin in hohen Ausbeuten erhalten.

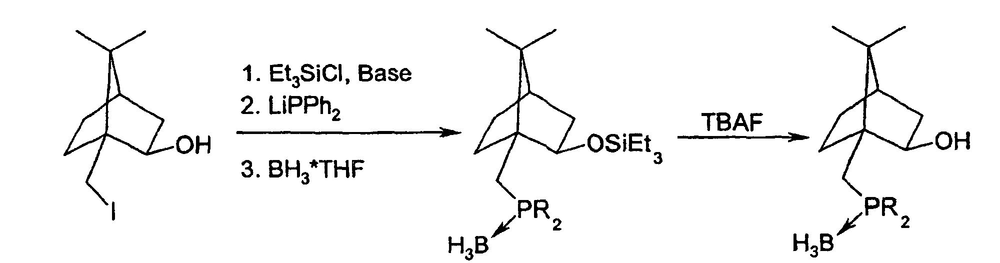

Die Einführung der zweiten Phosphineinheit gelingt durch Deprotonierung der Hydroxygruppe und Umsetzung mit einem Chlorphosphan, zur selktiven Einführung der Gruppe P(OₙR2)₂. Das Phosphin-Phosphinit kann ebenfalls mit einem Boran-THF-Addukt als Boran-Komplex abgefangen werden. Die Dekomplexierung erfolg durch Zugabe einer Stickstoffbase.

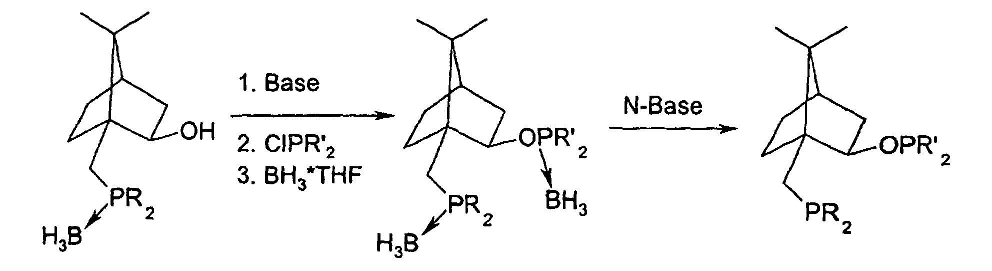

Zur Herstellung der Diphosphine werden die Phosphin-Phosphinite unter Inversion der Stereochemie thermisch zum Phosphinoxid in Lösung bei einer Temperatur zwischen 100°C und 200°C umgelagert. Eine nachfolgende Reduktion liefert das Diphosphin.

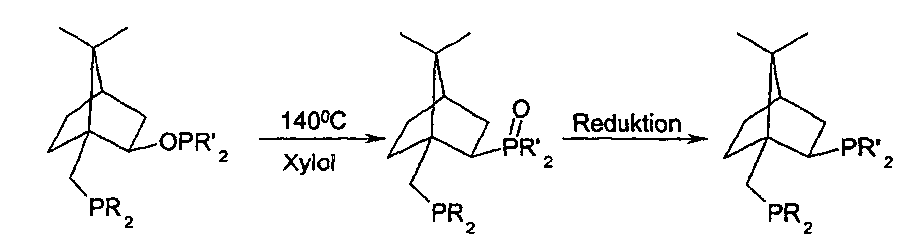

Die Verbindungen der allgemeinen Formel (I) können als Liganden an Metallen in asymmetrischen, Metall-katalysierten Reaktionen (wie z.B. Hydrierung, der Hydroformylierung, der Umlagerung, der allylischen Alkylierung, der Cyclopropanierung, Hydrosilylierung, Hydridübertragungen, Hydroborierungen, Hydrocyanierungen, Hydrocarboxylierungen, Aldol Reaktionen oder Heck-Reaktion) sowie bei Polymerisationen eingesetzt werden. Sie sind insbesondere für asymmetrische Reaktionen gut geeignet.

Geeignete Komplexverbindungen, insbesondere der allgemeinen Formel (II), enthalten erfindungsgemäße Verbindungen der Formel (I) als Liganden.

[MₓP_{y}L_{z}S_{q}]Aᵣ (II)

wobei in der allgemeinen Formel (II) Mein Übergangsmetallzentrum, L gleiche oder verschiedene koordinierende organische oder anorganische Liganden und P erfindungsgemäße bidentate Organophosphorliganden der Formel (I) darstellen, S koordinierende Lösungsmittelmoleküle und A Äquivalente aus nicht koordinierenden Anionen repräsentiert, wobei x 1 oder 2 ist, y eine ganze Zahl größer oder gleich 1 ist und z, q und r unabhängig voneinander ganze Zahlen größer oder gleich 0 sind.

Die Summe y + z + q wird durch die an den Metallzentren zur Verfügung stehenden Koordinationszentren nach oben begrenzt, wobei nicht alle Koordinationsstellen besetzt sein müssen. Bevorzugt sind Komplexverbindungen mit oktaedrischer, pseudo-oktaedrischer, tetraedrischer, pseudo-tetraedrischer, quadratisch-planarer Koordinationssphäre, die auch verzerrt sein kann, um das jeweilige Übergangsmetallzentrum. Die Summe y + z + q ist in solchen Komplexverbindungen kleiner oder gleich 6x.

Die erfindungsgemäßen Komplexverbindungen enthalten mindestens ein Metallatom oder -ion, vorzugsweise ein Übergangsmetallatom oder -ion, insbesondere aus Palladium, Platin, Rhodium, Ruthenium, Osmium, Iridium, Kobalt, Nickel, oder/und Kupfer.

Bevorzugt sind Komplexverbindungen mit weniger als vier Metallzentren, besonders bevorzugt solche mit ein oder zwei Metallzentren. Die Metallzentren können dabei mit verschiedenen Metallatomen und/oder -ionen besetzt sein.

Bevorzugte Liganden L solcher Komplexverbindungen sind Halogenid, besonders Cl, Br und I, Dien, besonders Cyclooctadien, Norbornadien, Olefin, besonders Ethylen und Cycloocten, Acetato, Trifluoracetato, Acetylacetonato, Allyl, Methallyl, Alkyl, besonders Methyl und Ethyl, Nitril, besonders Acetonitril und Benzonitril, sowie Carbonyl und Hydrido Liganden.

Bevorzugte koordinierende Lösungsmittel S sind Amine, besonders Triethylamin, Alkohole, besonders Methanol und Aromaten, besonders Benzol und Cumol.

Bevorzugte nichtkoordinierende Anionen A sind Trifluoracetat, Trifluormethansulfonat, BF₄; ClO₄, PF₆, SbF₆, und BAr₄.

In den einzelnen Komplexverbindungen können dabei unterschiedliche Moleküle, Atome oder Ionen der einzelnen Bestandteile M, P, L, S und A enthalten sein.

Bevorzugt unter den ionisch aufgebauten Komplexverbindungen sind Verbindungen des Typs [RhP(Dien)]⁺A⁻, wobei P einen erfindungsgemäßen Liganden der Formel (I) repräsentiert.

Die Herstellung dieser Metall-Ligand-Komplexverbindungen kann in situ durch Reaktion eines Metallsalzes oder eines entsprechenden Vorkomplexes mit den Liganden der allgemeinen Formel (I) erfolgen. Darüber hinaus kann eine Metall-Ligand-Komplexverbindung durch Reaktion eines Metallsalzes oder eines entsprechenden Vorkomplexes mit den Liganden der allgemeinen Formel (I) und anschließende Isolierung gewonnen werden. Die Erzeugung einer solchen Komplexverbindung erfolgt bevorzugt in einer Eintopfreaktion unter Rühren bei erhöhter Temperatur. Katalytisch aktive Komplexverbindungen können dabei auch direkt im Reaktionsansatz der geplanten katalytischen Umsetzung erzeugt werden werden.

Beispiele für die Metallsalze sind Metallchloride, -bromide, -iodide, -cyanide, -nitrate, - acetate, -acetylacetonate, -hexafluoracetylacetonate, tetrafluoroborate, - perfluoracetate oder -triflate, insbesondere des Palladium, Platins, Rhodium, Ruthenium, Osmium, Iridium, Kobalts, Nickels oder/und des Kupfers. Beispiele für die Vorkomplexe sind:
Cyclooctadienpalladiumchlorid, Cyclooctadienpalladiumiodid, 1,5-Hexadienpalladiumchlorid, 1,5-Hexadienpalladiumiodid, Bis(dibenzylidenaceton)palladium, Bis(acetonitril)palladium(II)chlorid, Bis(acetonitril)palladium(II)bromid, Bis(benzonitril)palladium(II)chlorid, Bis(benzonitril)palladium(II)bromid, Bis(benzonitril)palladium(II)iodid, Bis(allyl)palladium, Bis(methallyl)palladium, Allylpalladiumchlorid-Dimer, Methallylpalladiumchlorid-Dimer, Tetramethylethylendiaminpalladiumdichlorid, Tetramethylethylendiaminpalladiumdibromid, Tetramethylethylendiaminpalladiumdiiodid, Tetramethylethylendiaminpalladiumdimethyl,
Cyclooctadienplatinchlorid, Cyclooctadienplatiniodid, 1,5-Hexadienplatinchlorid, 1,5-Hexadienplatiniodid, Bis(cyclooctadien)platin, Kalium(ethylentrichloroplatinat), Cyclooctadienrhodium(I)chlorid-Dimer, Norbomadienrhodium(I)chlorid-Dimer, 1,5-Hexadienrhodium(I)chlorid-Dimer, Tris(triphenylphosphan)rhodium(I)chlorid, Hydridocarbonyltris(triphenylphosphan)rhodium(I)chlorid, Bis(cyclooctadien)rhodium(I)perchlorat, Bis(cyclooctadien)rhodium(I)tetrafluorborat, Bis(cyclooctadien)rhodium(I)triflat, Bis(acetonitrilcyclooctadien)rhodium(I)perchlorat, Bis(acetonitrilcyclooctadien)rhodium(I)tetrafluorborat, Bis(acetonitrilcyclooctadien)rhodium(I)triflat,
Cyclopentadienrhodium(III)chlorid-Dimer, Pentamethylcyclopentadienrhodium(III)chlorid-Dimer, (cyclooctadien)Ru(η³-allyl)₂, ((cyclooctadien)Ru)₂(acetat)₄, ((Cyclooctadien)Ru)₂(trifluoracetat)₄, RuCl₂(Aren)-Dimer, Tris(triphenylphosphan)ruthenium(II)chlorid, Cyclooctadienruthenium(II))chlorid, OsCl₂(Aren)-Dimer, Cyclooctadieniridium(I)chlorid-Dimer, Bis(cycloocten)iridium(I)chlorid-Dimer, Bis(cyclooctadien)nickel, (Cyclododecatrien)nickel, Tris(norbornen)nickel, Nickeltetracarbonyl, Nickel(II)acetylacetonat, (Aren)kupfertriflat, (Aren)kupferperchlorat, (Aren)kupfertrifluoracetat, Kobaltcarbonyl.

Die Komplexverbindungen auf Basis von ein oder mehreren Metallen der metallischen Elemente, insbesondere aus der Gruppe von Ru, Co, Rh, Ir, Ni, Pd, Pt, Cu, können bereits Katalysatoren sein oder zur Herstellung von Katalysatoren auf Basis eines oder mehrerer Metalle der metallischen Elemente, insbesondere aus der Gruppe von Ru, Co, Rh, Ir, Ni, Pd, Pt, Cu verwendet werden. Alle diese Komplexverbindungen sind besonders geeignet in der asymmetrischen Hydrierung von C=C-, C=O- oder C=N-Bindungen, in denen sie hohe Aktivitäten und Selektivitäten aufweisen und in der asymmetrischen Hydroformylierung. Insbesondere erweist es sich hier als vorteilhaft, daß sich die Liganden der allgemeinen Formel (I) durch ihre einfache, breite Abwandelbarkeit sterisch und elektronisch sehr gut auf das jeweilige Substrat und die katalytische Reaktion abstimmen lassen.

Entsprechende Katalysatoren enthalten mindestens eine der erfindungsgemäßen Komplexverbindungen.

### Ausführungsbeispiele :

### Allgemeines

Reaktionen luftempfindlicher Verbindungen wurden in einer argongefüllten Glove-Box oder in Standard Schlenkrohren durchgeführt. Lösungsmittel Tetrahydrofuran (THF), Diethylether und Dichlormethan wurden entgast und mittels einer Lösungsmitteltrocknungsanlage (Innovative Technologies) durch Filtration durch eine mit aktiviertem Aluminiumoxid gefüllte Säule absolutiert, Toluol und Pentan wurden zusätzlich durch eine mit einem Kupferkatalysator gefüllte Säule von Sauerstoff befreit.

Die folgenden Beispiele dienen zur Erläuterung der Erfindung. Sie sollen in keiner Weise eine Beschränkung darstellen.

### Beispiel 1: (1S)-10-lodocampher

Zu einer Lösung von 40 mmol (1S)-Campher-10-sulfonsäure und 200 mmol Triphenylphosphin in 400 ml Toluol werden 120 mmol lod zugegeben und die Lösung 15 h unter Rückfluß erhitzt. Nach dem Abkühlen auf Raumtemperatur wird die Lösung zweimal mit 100 ml Wasser gewaschen und getrocknet. Das Rohprodukt wird nach dem Entfernen des Lösungsmittels mittels Chromatogrphie gereinigt und das Produkt in 85% Ausbeute erhalten.
¹H-NMR (CDCl₃):
δ = 0.90 (s, 3H), 1.08 (s, 3H), 1.46-1.33 (m, 1 H), 1.67-1.55 (m, 6H), 1.91 (d, 1 H), 2.06-1.95 (m, 2H), 2.16 (dd, 1 H), 2.40 (ddd, 1 H), 3.12 (d, 1 H), 3.31 (d, 1 H) ppm.

### Beispiel 2: (1S,2R)-10-lodo-isoborneol

Zu einer im Eisbad gekühlten Suspension von 17.0 mmol Lithiumaluminiumhydrid in 20 ml Ether werden tropfenweise 24.4 10-lodocampher in 60 ml Ether zugegeben. Die Reaktionsmischung wird 2 h im Eisbad und anschließend weitere 2 bei Raumtemperatur gerührt. Die Reaktionsmischung wird unter Eiskühlung hydrolysiert und die organische Phase abgetrennt. Die wäßrige Phase wird dreimal mit Ether und dreimal mit Dichlormethan extrahiert, die vereinigten organischen Phasen werden mit Natriumchlorid-Lösung gewaschen, getrocknet und das Lösungsmittel im Vakuum entfernt. Nach der Reinigung mittels Chromatographie wird das Produkt in 84% Ausbeute erhalten.
¹H-NMR (CDCl₃):
δ = 0.86 (s, 3H), 1.06 (m, 4H), 1.26 (ddd, 1 H), 1.54 (ddd, 1 H), 1.77-1.66 (m ,2H), 1.83 (ddd, 1H), 2.02 (dd, 1 H), 2.12 (d, 1 H), 3.18 (d, 1 H), 3.45 (d, 1 H), 3.78 (ddd, 1 H) ppm.

### Beispiel 3: (1S,2R)-2-Triethylsiloxy-10-iodobornan

Zu einer gekühlten Lösung von 7.14 mmol 10-lodo-isoborneol und 8.52 mmol Imidazol in 10 DMF werden tropfenweise 7.83 mmol Triethylchlorosilan gegeben und die Lösung 15 min im Eisbad gerührt, anschließend wird weitere 14 h bei Raumtemperatur gerührt. Das Reaktionsgemisch wird mit Wasser hydrolysiert, mit Dichlormethan verdünnt und die wäßrige Phase anschließend viermal mit Dichlormethan extrahiert. Nach Trocknen und Reinigung wird das Produkt mit 96% Ausbeute erhalten.
¹H-NMR (CDCl₃):
δ = 0.52 (q, 2H), 0.64 (q, 4H), 0.86 (s, 3H), 0.93 (t, 3H), 0.98 (t, 6H), 1.01-0.95 (m 1H), 1.04 (s, 3H), 1.22 (ddd, 1 H), 1.51 (m, 3H), 1.80-1.73 (m, 1H), 2.01 (dd, 1H), 3.14 (d, 1 H), 3.49 (d, 1H), 3.74 (dd, 1 H), 3.49 (d, 1 H), 3.74dd, 1H) ppm.

### Beispiel 4: (1S,2R)-10-Boranatodiphenytphophino-2-trimethylsilyloxybornan

Zu einer gekühlten Lösung von Lithiumdiphenylphosphin in 6 ml THF wird tropfenweise 2-Triethylsiloxy-10-iodobornan zugegeben und weitere 30 min im Eisbad gerührt. Anschließend wird die Lösung 30 min bei Raumtemperatur gerührt und 1 h unter Rückfluß erhitzt. Nach dem Abkühlen auf 0°C werden 5.80 mmol Boran-THF Addukt zugeben und weitere 60 min gerührt. Die Reaktion wird durch die Zugabe von Wasser beendet, die organische Phase wird abgetrennt und die wäßrige Phase wird dreimal mit Dichlormethan extrahiert. Die organische Phase wird getrocknet und das Lösungsmittel im Vakuum entfernt. Nach der Reinigung mittels Chromatographie wird das Produkt mit 80% Ausbeute erhalten.
¹H-NMR (CDCl₃):
δ = 0.70 (q, 6H), 0.75 (s, 3H), 0.89 (ddd, 1 H), 0.97 (t, 9H), 1.02 (s, 3H), 1.06 (dd, 1 H), 1.17 (ddd, 1 H), 1.80-0.5 (br, BH₃), 1.48-1.37 (m, 1 H), 1.61 (dd, 1 H), 1.66 (dd, 1H), 1.76 (ddd, 1 H), 2.21 (dd, 1H), 2.83 (dd, 1 H), 4.09 (dd, 1 H), 7.53-7.31 (m, 6H), 7.62-7.54 (m, 2H), 7.89-7.82 (m,2H)
³¹P- NMR (CDCl₃):
δ = 12.7 (d, 1P) ppm.

### Beispiel 5: (1S,2R)-10- (Boranatodiphenylphosphino)isobomeol

Zu einer Lösung von 4.39 mmol 10-Boranatodiphenylphophino-2-trimethylsilyloxyboran in 4 ml THF werden 11 mmol Tetrabutylammoniumfluorid gegeben und die Lösung 90 min bei Raumtemperatur gerührt. Anschließend werden Wasser und Dichlormethan zugesetzt, die organische Phase abgetrennt und die wäßrige Phase dreimal mit Dichlormethan extrahiert. Nach dem Trocknen und Einengen des Lösungsmittels wird das Rohprodukt mittels Chromatographie gereinigt. Das Produkt fällt als farbloser Feststoff mit einer Ausbeute von 94 % an. ¹H-NMR (CDCl₃):
δ = 0.59 (ddd, 1 H), 0.78 (s, 3H), 0.94-0.80 (m, 2H), 1.08 (s, 3H), 1.50-1.37 (m, 1 H), 1.60-0.75 (br, BH₃), 1.63 (dd, 1H), 1.68 (dd, 1H), 1.78 (ddd, 1 H), 2.33 (dd, 1 H), 2.58 (dd, 1 H), 3.10 (br, 1 H), 4.14 (dd, 1H), 7.56-7.37 (m, 6H), 7.64-7.57 (m, 2H), 7.89-7.81 (m, 2H) ppm.
³¹P- NMR (CDCl₃):
δ = 10.4 (d, 1P) ppm.

### Beispiel 6: (1S,2R)-10-Boranato-diphenylphosphino-2-boranatodiphenylphosphinoxy-bornan

Zu einer auf -78°C gekühlten Lösung von 0.28 mmol 10-(Boranatodiphenylphosphino)-isoborneol in 4 ml THF wird tropfenweise n-Butyllithium zugegeben und 1h gerührt. Zu dieser Lösung werden 0.34 mmol Chlordiphenylphosphin gegeben, 2h bei -78°C und weitere 7h Raumtemperatur gerührt, anschließend wird die Lösung 2h unter Rückfluß erhitzt. Die Reaktionslösung wird auf 0°C abgekühlt. Zu dieser Lösung werden 0.60 mmol Boran-THF Addukt gegeben und 1 h bei 0°C gerührt. Die Reaktion wird durch Zugabe von Wasser beendet. Die wäßrige Phase wird dreimal mit Dichlormethan extrahiert. Nach dem Trocknen und Einengen wird das Rohprodukt mittels Chromatographie gereinigt und das Produkt in einer Ausbeute von 53% erhalten.
¹H-NMR (CDCl₃):
δ = 0.93-0.83 (m, 5H), 1.06 (s, 3H), 1.53-1.44 (m, 1 H), 1.70-0.75 (Br, BH3), 1.70-1.57 (m, 3H), 1.88 (ddd, 1 H), 2.26 (dd, 1H), 2.79 (dd, 1H), 4.49 (ddd, 1H), 7.82-7.29 (m, 20H) ppm.
³¹P- NMR (CDCl₃):
δ = 12.6 (s, 1P), 102.6 (d, 1P) ppm.

### Beispiel 7: (1S,2R)-10-Diphenylphosphino-2-diphenylphosphinit-boman

Es werden 2.72 mmol (1S,2R)-10-Boranato-diphenylphosphino-2-boranatodiphenylphosphinoxy-bornan in 75 ml Toluol gelöst und mit 10.8 mmol DABCO versetzt. Die Lösung wird 16h auf 85°C erhitzt und anschließend das Lösungsmittel im Vakuum entfernt. Das Produkt wird in Ether/Pentan (1:3) aufgenommen, über neutrales Aluminiumoxid filtriert und das Lösungsmittel im Vakuum entfernt. Das Produkt wird in einer Ausbeute von 67% erhalten.
¹H-NMR (CDCl₃):
δ = 0.72 (s, 3H), 0.80 (m, 2H), 0.99 (m, 1H), 1.10 (s, 3H), 1.36 (m 1 H), 1.57 (m, 2H) 1.81 (m, 1 H), 2.13 (dd, 1 H), 2.27 (dd, 1H), 4.32 (ddd, 1H), 6.70-7.09 (m, 8H), 7.22-7.24 (m 6H), 7.39-7.46 (m, 6H) ppm.
³¹P- NMR (CDCl₃):
δ = -24.0 (1P), 112.2 (1P) ppm.

### Beispiel 8: (1S,2R)-10-Diphenylphosphino-2-bis-(3,5-dimethyl-phenyl)phosphinitboman

Die Verbindung wurde analog zu Beispiel 1-7 dargestellt und das Produkt wird in einer Ausbeute von 61% erhalten.
¹H-NMR (CDCl₃):
δ = 0.82 (s, 3H), 1.33-0.84 (m, 15H), 1.12(s, 3H), 2.79 (s, 12H), 4.32 (ddd, 1H), 7.62-6.81 (m, 16H) ppm.
³¹P- NMR (CDCl₃):
δ = -24.1 (1P), 112.6 (1 P) ppm.

### Beispiel 9: (1S,2R)-10-Diphenylphosphino-2-dicyclohexylphosphinit-bornan

Die Verbindung wurde analog zu Beispiel 1-7 dargestellt und das Produkt wird in einer Ausbeute von 55% erhalten.
¹H-NMR (CDCl₃):
δ = 0.73 (s, 3H), 0.99 (s, 3H), 2.01-0.76 (m, 29H), 2.08 (dd,1 H), 2.34 (dd,1H), 4.39 (m, 1 H), 7.21-7.14 (m, 10H) ppm.
³¹P- NMR (CDCl₃):
δ = -25.6 (1 P), 143.2 (1P) ppm.

### Beispiel 10: (1S,2R)-6-{1-[(Diphenylphosphanyl)-methyl]-7,7-dimethylbicyclo[2.2.1]hept-2-yloxy}-5,7-dioxa-6-phospha-dibenzo[a,c]cyclohepten

Die Verbindung wurde analog zu Beispiel 1-7 dargestellt und das Produkt wird in einer Ausbeute von 48% erhalten.
¹H-NMR (CDCl₃):
δ = 0.74(s, 3H), 0.81(m, 2H), 0.99 (m, 1H), 1.07s, 3H), 1.37(m 1H), 1.59(m, 2H) 1.81 (m, 1 H), 2.11 (dd, 1 H), 2.27 (dd, 1H), 4.66 (ddd, 1 H), 6.83 (m, 1 H), 7.13-7.17 (m 7H), 7.23-7.38 (m, 10H), 7.58 (m, 2H) ppm.
³¹P- NMR (CDCl₃): δ = -24.5 (1 P), 151.4 (1 P) ppm.

### Hydrierbeispiele

Allgemeine Arbeitsvorschrift zur Hydrierung von Acetamidozimtsäuremethylester und Acetamidoacrylsäuremethylester

Es werden 0.6 µmol Rh(COD)₂OTf und 0.66 µmol Ligand 10 min in 1 ml Methanol gerührt. Zu dieser Lösung werden 300 µmol Acetamidozimtsäuremethylester bzw. Acetamidoacrylsäuremethylester (in 1 ml Methanol) dosiert. Bei Raumtemperatur und unter 5 bar Wasserstoffatmosphäre wird die Reaktionsmischung 2h im Autoklaven gerührt. Das Reaktionsgemisch wird über Kieselgel filtriert und aus dem Rohprodukt wird Enantiomerenüberschuß mittels HPLC bestimmt.

Allgemeine Arbeitsvorschrift zur Hydrierung von N-Acetyl-2phenyl-1-ethenylamin Es werden 0.6 µmol Rh(COD)₂OTf und 0.66 µmol Ligand 10 min in 1 ml Methanol gerührt. Zu dieser Lösung werden 300 µmol N-Acetyl-2phenyl-1-ethenylamin (in 1 ml Methanol) dosiert. Bei 40°C und unter 10 bar Wasserstoffatmosphäre wird die Reaktionsmischung 2h im Autoklaven gerührt. Das Reaktionsgemisch wird über Kieselgel filtriert und aus dem Rohprodukt wird Enantiomerenüberschuß mittels HPLC bestimmt.

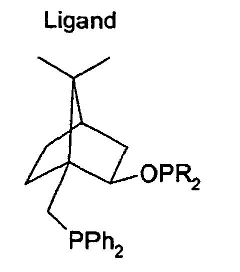

| R | Acetamidozimtsäuremethylester (%ee) | N-Acetyl-2phenyl-1-ethenylamin (%ee) | Acetamidoacryl-säuremethylester (%ee) |
|---|---|---|---|
| Ph | 46 | -16 | -88 |
| Cy | -3 | -89 | 6 |
| 3,5-Me-Ph | -18 | 7 | -94 |

## Patentansprüche

1. Bidentate Organophosphorliganden der allgemeinen Formel (I), 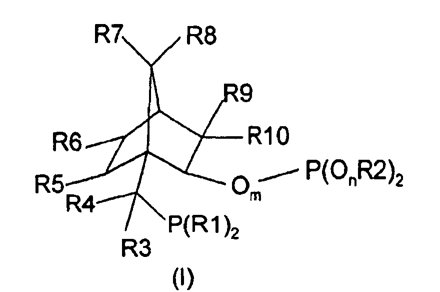 worin
m und n unabhängig voneinander 0 oder 1 sein können und
R1-R2, unabhängig voneinander für Reste aus der Gruppe der C₁-C₂₄ Alkyl-, C₃-C₈ Cycloalkyl-, wobei ein oder zwei der Kohlenstoffatome des Cyclus durch Heteroatome, ausgewählt aus der Gruppe N, O, S, ersetzt sein können, C₂-C₁₃ Heteroaryl-, wobei die Zahl der Heteroatome, ausgewählt aus der Gruppe N, O, S, 1-4 betragen kann, C₆-C₁₄ Aryl-, Phenyl-, Naphthyl-, Fluorenyl-Reste stehen,
wobei die vorgenannten Reste selbst jeweils ein- oder mehrfach unabhängig voneinander mit Substituenten ausgewählt aus der Gruppe der Wasserstoff-, C₁-C₂₀ Alkyl-, C₂-C₂₀ Alkenyl-, C₁-C₁₀ Haloalkyl-, C₃-C₈ Cycloalkyl-, C₂-C₉ Heterocycloalkyl-, wobei die Zahl der Heteroatome, aus der Gruppe N, O, S, 1-4 betragen kann, C₆-C₈ Aryl,-Phenyl-, Naphthyl-, Fluorenyl-, C₂-C₆ Heteroaryl-, wobei die Zahl der Heteroatome, aus der Gruppe N, O, S, 1-4 betragen kann, C₁-C₁₀ Alkoxy-, C₁-C₉ Trihalomethylalkyl-, Halogeno-, Hydroxy-, Trifluormethylsulfonato-, Oxo-, Amino-, C₁-C₈ substituierte Amino- der Formen NH-Alkyl-C₁-C₈, NH-Aryl-C₅-C₆, N-Alkyl₂-C₁-C₈, N-Aryl₂-C₅-C₆, N-Alkyl₃-C₁-C₈⁺, N-Aryl₃-C₅-C₆⁺, NH-CO-Alkyl-C₁-C₈, NH-CO-Aryl-C₅-C₆, Cyano-, Carboxylato- der Formen COOH und COOQ wobei Q entweder ein einwertiges Kation oder C₁-C₈-Alkyl darstellt, C₁-C₆-Acyloxy-, Sulfinato-, Sulfonato- der Formen SO₃H und SO₃Q wobei Q entweder ein einwertiges Kation, C₁-C₈-Alkyl oder C₆-Aryl darstellt, Phosphato- der Formen PO₃H₂, PO₃HQ und PO₃Q₂ wobei Q entweder ein einwertiges Kation, C₁-C₈-Alkyl oder C₆-Aryl darstellt, Tri- C₁-C₆ Alkylsilylreste substituiert sein können,
wobei die beiden Reste R1 oder die beiden Reste R2 miteinander verbrückt sein können,
wobei der entstehende Zyklus mit linearen oder verzweigten C₁-C₁₀ Alkyl-, C₆-Aryl-, Benzyl-, C₁-C₁₀ Alkoxy-, Hydroxy- oder Benzyloxygruppen, substituiert sein kann, und worin
R3-R 10 unabhängig voneinander ein Wasserstoffatom oder eine C₁-C₂₄Alkyl-, C₁-C₁₀-Haloalkyl-, C₃-C₈ Cycloalkyl-, C₃-C₈ Cycloalkenyl-, wobei ein oder zwei Kohlenstoffatome des Cycloalkyls oder Cycloalkenyls auch durch Heteroatome ausgewählt aus der Gruppe N, O, S, ersetzt sein können, C₆-C₁₄ Aryl-, Phenyl-, Naphthyl-, Fluorenyl-, C₂-C₁₃ Heteroaryl-Gruppe, wobei die Zahl der Heteroatome, ausgewählt aus der Gruppe N, O, S, 1-4 betragen kann, sein können,
wobei die vorgenannten Gruppen selbst jeweils ein- oder mehrfach unabhängig voneinander mit Wasserstoff, C₁-C₂₀ Alkyl-, C₂-C₂₀ Alkenyl-, C₁-C₁₀ Haloalkyl-, C₃-C₈ Cycloalkyl-, C₃-C₈ Cycloalkenyl-, C₂-C₉ Heteroalkyl-, C₁-C₉ Heteroalkenyl-, C₆-C₈Aryl-, Phenyl-, Naphthyl-, Fluorenyl-, C₂-C₆ Heteroaryl-, wobei die Zahl der Heteroatome, insbesondere aus der Gruppe N, O, S, 1-4 betragen kann, C₁-C₁₀ Alkoxy-, Trifluormethyl-, Fluoro-, Oxo-, Amino-, C₁-C₈ substituierte Amino-Resten der Formen N-Alkyl₂-C₁-C₈, N-Aryl₂-C₅-C₆, N-Alkyl₃-C₁-C₈⁺, N-Aryl₃-C₅-C₆⁺ substituiert sein können,
und wobei zwei dieser Substituenten auch verbrückt sein können, und worin
P ein dreiwertiger Phosphor ist.

2. Verbindung nach Anspruch 1, **dadurch gekennzeichnet, daß** R7 und R8 für Methyl und R3-R6 und R9-R10 für Wasserstoff stehen.

3. Verbindungen nach Anspruch 1 oder 2, **dadurch gekennzeichnet, daß** die Substituenten R1 und R2 unabhängig voneinander für 1-Methylethyl, Cyclohexyl, Cyclopentyl, Phenyl, 2-Methyl-phenyl, 3,5-Dimethyl-phenyl, 4-Methyl-phenyl, 4-Methoxy-phenyl, 3,5-Bis-(trifluormethyl)-phenyl, 4-Trifluormethyl-phenyl, 3,5-Dimethyl-4-Methoxy-phenyl, 4-Phenoxyl, 4-Dialkyamino, 2-Alkylphenyl, 3-Alkylphenyl, 4-Alkylphenyl, 2,6-Dialkylphenyl, 3,5-Dialkylphenyl, 3,4,5-Trialkylphenyl, 2-Alkoxyphenyl, 3-Alkoxyphenyl, 4-Alkoxyphenyl, 2,6-Dialkoxylphenyl, 3,5-Dialkoxyphenyl, 3,4,5-Triialkoxyphenyl, 3,5-Dialkyl-4-Alkoxyphenyl, 3,5-Dialkyl-4-dialkylaminophenyl, 4-Dialkylamino, 3,5-Trifluormethyl, 4-Trifluormethyl, 2-Sulfonyl, 3-Sulfonyl, 4-Sulfonyl, ein bis vierfach halogenierte Phenyl und Naphtyl stehen.

4. Verbindungen nach Anspruch 1 und 2, **dadurch gekennzeichnet, daß** die Verbindungen der Formel (I) enantiomerenangereichert sind.

5. Verbindungen nach Anspruch 4, **dadurch gekennzeichnet, daß** die Enantiomerenanreicherung 90 % übersteigt.

6. Komplexverbindungen erhältlich durch Zusammengeben eines Übergangsmetallsalzes oder eines Übergangsmetallvorkomplexes mit bidentaten Organophosphorliganden der Formel (I).

7. Komplexverbindungen der allgemeinen Formel (II)
[MₓP_{y}L_{z}S_{q}]Aᵣ (II)
wobei in der Formel (II)
M ein Übergangsmetallzentrum
L gleiche oder verschiedene koordinierende organische oder anorganische Liganden,
S koordinierende Lösungsmittelmoleküle und
A Äquivalente aus nicht koordinierenden Anionen repräsentiert,
wobei x 1 oder 2 ist, y eine ganzen Zahlen größer oder gleich 1 ist, z, q und r ganze Zahlen größer oder gleich 0 sind, wobei die Summe y + z + q durch die an den Metallzentren zur Verfügung stehenden Koordinationszentren nach oben begrenzt ist, wobei aber nicht alle Koordinationsstellen besetzt sein müssen,
**dadurch gekennzeichnet, daß** P für erfindungsgemäße bidentate Organophosphorliganden der Formel (I) steht.

8. Komplexverbindung gemäß Anspruch 6 oder 7 **dadurch gekennzeichnet, daß** die Komplexverbindung ein Metall ausgewählt aus der Gruppe Ru, Co, Rh, Ir, Ni, Pd, Pt, Cu enthält.

9. Verfahren zur Herstellung von bidentaten Phosphin-Phosphonit-Verbindung gemäß Formel (I) mit n gleich 1 umfassend folgende Verfahrensschritte:
a) Substitution des 10-Sulfonsäurerestes eines 10-Camphersulfonsäure-Derivates durch einen Halogenidrest durch Zusammengeben eines 10-Camphersulfonsäure-Derivates und eines Phosphortrihalogenids oder eines molekularen Halogens in Gegenwart eines Phosphins,
b) Silylierung der resultierenden Hydroxygruppe des 10-Halogenocampher-Derivates,
c) Phospinierung des 10-Halogencampher-Derivates durch Substitution des Halogenrestes mit einer Phosphingruppe durch Zugabe eines Alkaliphosphins,
d) Abfangen des Zwischenproduktes durch Zugabe eines Boran-Adduktes,
e) Entfernung der Silylschutzgruppe,
f) basische Phosphinierung der Hydroxygruppe durch Zugabe eines Phosphinhalogenids und
g) Entfernung der Boranschutzgruppe durch Zugabe einer stickstoffhaltigen Base.

10. Verfahren zur Herstellung von bidentaten Diphosphin-Verbindung gemäß Formel (I) mit n gleich 0 ausgehend von Phosphin-Phosphiniten durch thermische Umlagerung der Phosphinitgruppe in das Phosphinoxid und anschließender Reduktion zum Diphosphin.

11. Verwendung einer Komplexverbindung nach einem der Ansprüche 6 bis 8 als Katalysator für asymmetrische Reaktionen oder Polymerisationen.

12. Verwendung einer Komplexverbindung nach einem der Ansprüche 6 bis 8 als Katalysator für asymmetrische Hydrierung, Hydroformylierung, Umlagerung, allylischen Alkylierung, Cyclopropanierung, Hydrosilylierung, Hydridübertragungsreaktionen, Hydroborierungen, Hydrocyanierungen, Hydrocarboxylierungen, Aldol Reaktionen, Pauson-Khand Reaktionen oder Heck-Reaktionen.

13. Verwendung einer Komplexverbindung nach einem der Ansprüche 6 bis 8 als Katalysator für die asymmetrische Hydrierung und/oder Hydroformylierung.

## Claims

1. A bidentate organophosphorus ligand of the formula (1 ), 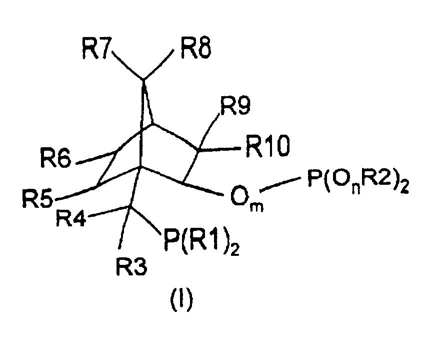 where
m and n may each be, independently of one another, 0 or 1 and
R1-R2 are, independently of one another, radicals selected from the group consisting of C₁-C₂₄-alkyl, C₃-C₈-cycloalkyl in which one or two of the carbon atoms of the ring may be replaced by heteroatoms selected from the group consisting of N, O and S. C₂-C₁₃-heteroaryl in which the number of heteroatoms selected from the group consisting of N, O and S may be 1-4, C₆-C₁₄-aryl, phenyl, naphthyl and fluorenyl radicals,
where the abovementioned radicals may themselves each be substituted by, independently of one another, one or more substituents selected from the group consisting of hydrogen, C₁-C₂₀-alkyl. C₂-C₂₀-alkenyl, C₁-C₁₀-haloalkyl, C₃-C₈-cycloalkyl, C₂-C₉-heterocycloalkyl, in which the number of heteroatoms selected from the group consisting of N, O and S may be 1-4, C₆-C₈-aryl, phenyl, naphthyl, fluorenyl, C₂-C₆-heteroaryl, in which the number of heteroatoms selected from the group consisting of N, O and S may be 1-4, C₁-C₁₀-alkoxy, C₁-C₉-trihalomethylalkyl, halo, hydroxy, trifluoromethylsulfonato, oxo, amino, C₁-C₈-substituted amino of the formulae NH-alkyl-C₁-C₈, NH-aryl-C₅-C₆, N-alkyl₂-C₁-C₈, N-aryl₂-C₅-C₆, N-alKyl₃-C₁-C₈⁺, N-aryl₃-C₅-C₆⁺, NH-CO-alkyl-C₁-C₈, NH-CO-aryl-C₅-C₆, cyano, carboxylato of the formulae COOH and COOQ, where Q is either a monovalent cation or C₁-C₈-alkyl, C₁-C₆-acyloxy, sulfinato, sulfonato of the formulae SO₃H and SO₃Q, where Q is either a monovalent cation, C₁-C₈-alkyl or C₆-aryl, phosphato of the formulae PO₃H₂, PO₃HQ and PO₃Q₂, where Q is either a monovalent cation, C₁-C₈-alkyl or C₆-aryl, tri-C₁-C₆-alkylsilyl radicals,
where the two radicals R1 or the two radicals R2 may be bridged,
where the resulting ring may be substituted by linear or branched C₁-C₁₀-alkyl, C₆-aryl, benzyl, C₁-C₁₀-alkoxy, hydroxy or benzyloxy groups, and where
R3-R10 may each be, independently of one another, a hydrogen atom or a C₁-C₂₄-alkyl, C₁-C₁₀-haloalkyl, C₃-C₈-cycloalkyl, C₃-C₈-cycloalkenyl group, where one or two carbon atoms of the cycloalkyl or cycloalkenyl may also be replaced by heteroatoms selected from the group consisting of N, O and S, C₆-C₁₄-aryl, phenyl, naphthyl, fluorenyl or C₂-C₁₃-heteroaryl group in which the number of heteroatoms selected from the group consisting of N, O and S may be 1-4,
where the abovementioned groups may themselves each be, independently of one another, monosubstituted or polysubstituted by hydrogen, C₁-C₂₀-alkyl, C₂-C₂₀-alkenyl, C₁-C₁₀-haloalkyl, C₃-C₈-cycloalkyl, C₃-C₈-cycloalkenyl, C₂-C₉-heteroalkyl, C₁-C₈-heteroalkenyl, C₆-C₈-aryl, phenyl, naphthyl, fluorenyl, C₂-C₆-heteroaryl in which the number of heteroatoms, in particular from the group consisting of N, O and S, may be 1-4, C₁-C₁₀-alkoxy, trifluoromethyl, fluoro, oxo, amino, C₁-C₈-substituted amino radicals of the formulae N-alkyl₂-C₁-C₈, N-aryl₂-C₅-C₆, N-alkyl₃-C₁-C₈⁺, N-aryl₃-C₅-C6⁺,
and where two of these substituents may also be connected, and where
P is trivalent phosphorus.

2. A compound as claimed in claim 1, wherein R7 and R8 are methyl and R3-R6 and R9-R10 are hydrogen.

3. A compound as claimed in claim 1 or 2, wherein the substituents R1 and R2 are each, independently of one another, 1-methylethyl, cyclohexyl, cyclopentyl, phenyl, 2-methylphenyl, 3,5-dimethylphenyl, 4-methylphenyl, 4-methoxyphenyl, 3,5-bis(trifluoromethyl)phenyl, 4-trifluoromethylphenyl, 3,5-dimethyl-4-methoxyphenyl, 4-phenoxyl, 4-dialkylamino, 2-alkylphenyl, 3-alkylphenyl, 4-alkylphenyl, 2,6-dialkylphenyl, 3,5-dialkylphenyl, 3,4,5-trialkylphenyl, 2-alkoxyphenyl, 3-alkoxyphenyl, 4-alkoxyphenyl, 2,6-dialkoxyphenyl, 3,5-dialkoxyphenyl, 3,4,5-trialkoxyphenyl, 3,5-dialkyl-4-alkoxyphenyl, 3,5-dialkyl-4-dialkylaminophenyl, 4-dialkylamino, 3,5-trifluoromethyl, 4-trifluoromethyl, 2-sulfonyl, 3-sulfonyl, 4-sulfonyl, monohalogenated to tetrahalogenated phenyl and naphthyl.

4. A compound of the formula (I) as claimed in claim 1 or 2 which is enantiomerically enriched.

5. A compound as claimed in claim 4, wherein the enantiomeric enrichment exceeds 90%.

6. A complex obtainable by mixing a transition metal salt or a transition metal precursor complex with bidentate organophosphorus ligands of the formula (I).

7. A complex of the formula (II)
[MₓP_{y}L_{z}S_{q}]Aᵣ (II)
where, in the formula (II),
M is a transition metal center,
L are identical or different coordinating organic or inorganic ligands,
S are coordinating solvent molecules and
A are equivalents of noncoordinating anions,
where x is 1 or 2, y is an integer greater than or equal to 1, z, q and r are integers greater than or equal to 0, where the upper limit on the sum y + z +q is imposed by the number of coordination centers available on the metal centers but not all coordination sites have to be occupied,
wherein P are bidentate organophosphorus ligands of the formula (I).

8. A complex as claimed in claim 6 or 7 which contains a metal selected from the group consisting of Ru, Co, Rh, Ir, Ni, Pd, Pt, Cu.

9. A process for preparing bidentate phosphine phosphinite compounds of the formula (I) with n = 1, which comprises the following process steps:
a) replacement of the 10-sulfonic acid radical of a 10-camphorsulfonic acid derivative by a halide radical by mixing a 10-camphorsulfonic acid derivative and a phosphorus trihalide or a molecular halogen in the presence of a phosphine,
b) silylation of the resulting hydroxy group of the 10-halocamphor derivative,
c) phosphination of the 10-halocamphor derivative by replacement of the halogen radical by a phosphine group by addition of an alkaline metal salt of a phosphine,
d) conversion of the intermediate into a borane complex by addition of a borane adduct,
e) removal of the protective silyl group,
f) basic phosphination of the hydroxy group by addition of a phosphine halide and
g) removal of the protective borane group by addition of a nitrogen-containing base.

10. A process for preparing bidentate diphosphine compounds of the formula (I) with n = 0 starting from phosphine phosphinites by thermal rearrangement of the phosphinite group to the phosphine oxide and subsequent reduction to the diphosphine.

11. The use of a complex as claimed in any of claims 6 to 8 as catalyst for asymmetric reactions or polymerizations.

12. The use of a complex as claimed in any of claims 6 to 8 as catalyst for asymmetric hydrogenation, hydroformylation, rearrangement, allylic alkylation, cyclopropanation, hydrosilylation, hydride transfer reactions, hydroborations, hydrocyanations, hydrocarboxylations, aldol reactions, Pauson-Khand reactions or Heck reactions.

13. The use of a complex as claimed in any of claims 6 to 8 as catalyst for asymmetric hydrogenation and/or hydroformylation.

## Revendications

1. Ligands organophosphorés bicoordinés de formule générale (I), 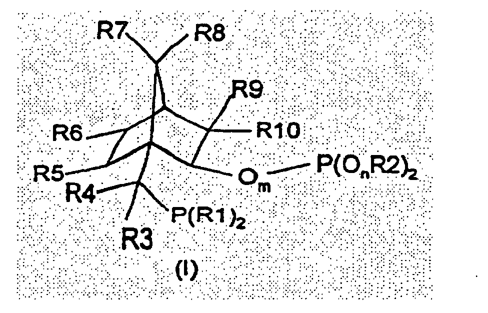 dans laquelle
m et n peuvent être indépendamment l'un de l'autre 0 ou 1 et
R1-R2 représentent indépendamment l'un de l'autre des groupes choisis parmi les groupes alkyle en C₁-C₂₄, cycloalkyle en C₃-C₈, dans lesquels un ou deux des atomes de carbone du cycle peuvent être remplacés par des hétéroatomes, choisis parmi N, O, S, hétéroaryle en C₂-C₁₃, dans lequel le nombre des hétéroatomes choisis parmi N, 0, S peut s'élever à 1-4, aryle en C₆-C₁₄, phényle, naphtyle, fluorényle,
dans lesquels les groupes précédents peuvent être substitués respectivement une ou plusieurs fois indépendamment les uns des autres par des substituants choisis parmi l'hydrogène, les groupes alkyle en C₁-C₂₀, alcényle en C₂-C₂₀, halogénoalkyle en C₁-C₁₀, cycloalkyle en C₃-C₈, hétérocycloalkyle en C₂-C₉, dans lesquels le nombre des hétéroatomes, choisis parmi N, O, S peut s'élever à 1-4, aryle en C₅-C₈, phényle, naphtyle, fluorényle, hétéroaryle en C₂-C₆, dans lesquels le nombre des hétéroatomes, choisis parmi N, O, S peut s'élever à 1-4, alcoxy en C₁-C₁₀, trihalogénométhylalkyle en C₁-C₉, halogéno, hydroxy, trifluorométhylsulfonato, oxo, amino, amino substitué en C₁-C₈ des formes NH-alkyle en C₁-C₈, NH-aryle en C₅-C₆, N-(alkyle en C₁-C₈)₂, N-(aryle en C₅-C₆)₂, N-(alkyle en C₁-C₈)₃⁺, N-(aryle en C₅-C₆)₃⁺, NH-CO-alkyle en C₁-C₈, NH-CO-aryle en C₅-C₆, cyano, carboxylato des formes COOH et COOQ dans laquelle Q représente soit un cation monovalent soit un groupe alkyle en C₁-C₈, acyloxy en C₁-C₆, sulfinato, sulfonato des formes SO₃H et SO₃Q dans laquelle Q représente soit un cation monovalent, un alkyle en C₁-C₈ ou aryle en C₆, phosphate des formes PO₃H₂, PO₃HQ et PO₃Q₂, dans lesquelles Q représente soit un cation monovalent, un alkyle en C₁-C₈ ou aryle en C₆, les groupes tri-alkyle en C₁-C₆-silyle, dans lesquels les deux groupes R1 ou les deux groupes R2 peuvent être pontés les uns avec les autres,
dans lesquels le cycle résultant peut être substitué par des groupes alkyle en C₁ à C₁₀ linéaires ou ramifiés, aryle en C₆, benzyle, alcoxy en C₁-C₁₀, hydroxy ou benzyloxy, et dans lesquels
R3-R10 peuvent être indépendamment les uns des autres un atome d'hydrogène ou un groupe alkyle en C₁-C₂₄, halogénoalkyle en C₁-C₁₀, cycloalkyle en C₃-C₈, cycloalcényle en C₃-C₈, dans lesquels un ou deux atomes de carbone du cycloalkyle ou cycloalcényle peuvent aussi être substitués par des hétéroatomes choisis dans le groupe N, O, S, aryle en C₆-C₁₄, phényle, naphtyle, fluorényle, fluorényle, hétéroaryle en C₂-C₁₃, dans lesquels le nombre des hétéroatomes, choisis dans le groupe N, O, S, peut s'élever à 1-4,
dans lesquels les groupes précédents peuvent être substitués respectivement une ou plusieurs fois indépendamment les uns des autres par l'hydrogène, des groupes alkyle en C₁-C₂₀, alcényle en C₂-C₂₀, halogénoalkyle en C₁-C₁₀, cycloalkyle en C₃-C₈, cycloalcényle en C₃-C₈, hétéroalkyle en C₂-C₉, hétéro-alcényle en C₁-C₉, aryle en C₆-C₈, phényle, naphtyle, fluorényle, hétéroaryle en C₂-C₆, dans lesquels le nombre des hétéroatomes, en particulier du groupe N, O, S peut s'élever à 1-4, alcoxy en C₁-C₁₀, trifluorométhyle, fluoro, oxo, amino, amino substitué en C₁-C₈ des formes N-(alkyle en C₁-C₈) ₂, N-(aryle en C₅-C₆)₂, N-(alkyle en C₁-C₈)₃⁺, N- ( aryle en C₅-C₆)₃⁺,
et dans lesquels deux de ces substituants peuvent aussi être pontés, et
dans laquelle
p est un phosphore trivalent.

2. Composé selon la revendication 1, **caractérisé en ce que** R7 et R8 représentent un méthyle et R3-R6 et R9-R10 représentent un hydrogène.

3. Composés selon la revendication 1 ou 2, **caractérisés en ce que** les substituants R1 et R2 représentent indépendamment l'un de l'autre un groupe 1-méthyléthyle, cyclohexyle, cyclopentyle, phényle, 2-méthyl-phényle, 3,5-diméthyl-phényle, 4-méthyl-phényle, 4-méthoxy-phényle, 3,5-bis-(trifluorométhyl)-phényle, 4-trifluorométhyl-phényle, 3,5-diméthyl-4-méthoxy-phényle, 4-phénoxyle, 4-dialkylamino, 2-alkylphényle, 3-alkylphényle, 4-alkylphényle, 2,6-dialkylphényle, 3,5-dialkylphényle, 3,4,5-trialkylphényle, 2-alcoxyphényle, 3-alcoxyphényle, 4-alcoxyphényle, 2,6-dialcoxyphényle, 3,5-dialcoxyphényle, 3,4,5-trialcoxyphényle, 3,5-dialkyl-4-alcoxyphényle, 3,5-dialkyl-4-dialkylaminophényle, 4-dialkylamino, 3,5-trifluorométhyle, 4-trifluorométhyle, 2-sulfonyle, 3-sulfonyle, 4-sulfonyle, phényle et naphtyle une à quatre fois halogéné.

4. Composés selon les revendications 1 et 2, **caractérisés en ce que** les composés de formule (I) sont enrichis en énantiomères.

5. Composés selon la revendication 4, **caractérisés en ce que** l'enrichissement en énantiomères dépasse 90 %.

6. Composés complexes pouvant être obtenus par mélange d'un sel de métal de transition ou d'un précurseur de complexe de métal de transition avec des ligands organophosphorés bicoordinés de formule (I).

7. Composés complexes de formule générale (II)
[MₓP_{y}L_{z}S_{q}]Aᵣ (II)
dans lesquels dans la formule (II)
M représente un centre de métal de transition
L représente des ligands coordinants organiques ou inorganiques identiques ou différents,
S représente des molécules de solvant coordinantes et
A représente des équivalents d'anions non coordinants,
dans lesquels x est 1 ou 2, y est un nombre entier supérieur ou égal à 1, z, q et r sont des nombres entiers supérieurs ou égaux à 0, dans lesquels la somme y + z + q est limitée vers le haut par les centres de coordination à disposition sur les centres métalliques, dans lesquels il ne faut cependant pas obligatoirement que toutes les positions de coordination soient occupées,
**caractérisés en ce que** P représente des ligands organophosphorés bicoordinés de formule (I) selon l'invention.

8. Composé complexe selon la revendication 6 ou 7 **caractérisé en ce que** le composé complexe contient un métal choisi dans le groupe Ru, Co, Rh, Ir, Ni, Pd, Pt, Cu.

9. Procédé pour la préparation de composé phosphine-phosphonite bicoordiné selon la formule (I) avec n égal à 1 comprenant les étapes suivantes de procédé :
a) substitution du groupe acide 10-sulfonique d'un dérivé d'acide 10-camphrosulfonique par un reste halogénure par mélange d'un dérivé d'acide 10-camphrosulfonique et d'un trihalogénure de phosphore ou d'un halogène moléculaire en présence d'une phosphine,
b) silylation du groupe hydroxy résultant du dérivé 10-halogénocamphre,
c) phosphination du dérivé 10-halogénocamphre par substitution du reste halogène par un groupe phosphine par ajout d'une phosphine de métal alcalin,
d) récupération du produit intermédiaire par ajout d'un adduit de borane,
e) élimination du groupe de protection silyle,
f) phosphination basique du groupe hydroxy par ajout d'un halogénure de phosphine et
g) élimination du groupe de protection borane par ajout d'une base azotée.

10. Procédé de préparation de composé diphosphine bicoordiné selon la formule (I) avec n égal 0 en partant de phosphine-phosphites par réarrangement thermique du groupe phosphinite en oxyde de phosphine et ensuite réduction en diphosphine.

11. Utilisation d'un composé complexe selon une des revendications 6 à 8 comme catalyseur pour des réactions asymétriques ou des polymérisations.

12. Utilisation d'un composé complexe selon une des revendications 6 à 8 comme catalyseur pour hydrogénation asymétrique, hydroformylation, réarrangement, alkylation asymétrique, cyclopropanation, hydrosilation, réactions de transfert hybride, hydroborations, hydrocyanations, hydrocarboxylations, réactions d'aldols, réactions de Pauson-Khand ou réactions de Heck.

13. Utilisation d'un composé complexe selon une des revendications 6 à 8 comme catalyseur pour l'hydrogénation asymétrique et/ou l'hydroformylation.
